# EUROPÄISCHE PATENTSCHRIFT

(11) **EP 2 371 412 B1**
(45) Veröffentlichungstag und Bekanntmachung des Hinweises auf die Patenterteilung: **31.07.2013**
(21) Anmeldenummer: 10158480.3
(22) Anmeldetag: 30.03.2010
(51) Int. Cl.: A61M 16/00

(54) **Beatmungs- oder Anästhesiesystem**
Ventilation or anaesthetic system
Système de ventilation ou d'anesthésie

(43) Veröffentlichungstag der Anmeldung: 05.10.2011
(60) Teilanmeldung: 13167921.9
(73) Patentinhaber: Dräger Medical GmbH, 23558 Lübeck (DE)
(72) Erfinder: Hansmann, Hans-Ulrich, 23858, Barnitz (DE); Eger, Marcus, Dr., 23562, Lübeck (DE); Glaw, Tobias, 23558, Lübeck (DE); Krüger, Thomas, 23858, Reinfeld (DE)
(74) Vertreter: UEXKÜLL & STOLBERG

(56) Entgegenhaltungen:
- WO-A1-98/48877
- WO-A1-2006/131149
- AU-B2- 707 148
- DE-A1- 10 164 446
- DE-B3-102007 062 214
- US-A- 5 353 788
- US-A1- 2006 152 197

## Beschreibung

Die vorliegende Erfindung betrifft eine Beatmungs- oder Anästhesiesystem gemäß dem Oberbegriff des Anspruches 1.

Für verschiedene medizinische Anwendungen, z. B. bei Operationen, ist eine Beatmung von Patienten notwendig. Beatmungsgeräte bzw. Beatmungssysteme dienen zur Beatmung von Patienten und können zusätzlichen mit einem Anästhesiemittelreflektor und Anästhesiemitteldosierer als Anästhesiegeräte bzw. Anästhesiesysteme auch zur Narkose eingesetzt werden. Bei einigen Beatmungs- oder Anästhesiesystemen kann wenigstens teilweise das von dem Patienten ausgeatmete Exspirationsgas wieder als Inspirationsgas wiederverwendet werden, d. h. es handelt sich um ein Rückatemsystem mit einem Atemluftkreissystem. In dem Beatmungsgerät mit dem Atemluftkreissystem ist eine Gasfördereinrichtung vorhanden, welche die Atemluft während der Inspiration zum Patienten leitet. Während und nach der Ausatmung ist die Gasfördereinrichtung entweder abgeschaltet oder wird nur mit einem sehr geringen Förderstrom betrieben.

Die von dem Beatmungs- oder Anästhesiesystem durchgeführte Beatmung kann entweder vollständig die Eigenbeatmungstätigkeit des Patienten übernehmen oder bei einem unterstützenden Verfahren die eigenen Atemtätigkeit des Patienten lediglich nur teilweise ersetzten, so dass die Atemmuskulatur des Patienten einen Teil der Atemtätigkeit übernimmt. Für eine verbesserte Therapie, insbesondere bei COPD-Patienten, ist eine Steuerung des Anteils der Beatmung erforderlich, d. h. der Anteil der Eigenbeatmungstätigkeit des Patienten kann gesteuert und/oder geregelt werden. Hierzu sind die Leistung der Gasfördereinheit des Beatmungs- oder Anästhesiesystems zu steuern und der Zeitpunkt und die Amplitude der Beatmung in Bezug auf die der Eigenatmung des Patienten anzupassen. Dazu wird mit elektromyographischen Sensoren (EMG-Sensoren) invasiv die elektromyographische Aktivität der Atemmuskulatur des Patienten erfasst. Die EMG-Sensoren sind invasiv, insbesondere in einer Luftröhre, am Patienten angeordnet.

Die DE 10 2007 062 214 B3 zeigt ein Verfahren zum automatischen Steuern eines Beatmungssystems für proportional unterstützende Ventilation mit einer Steuereinrichtung und einem Ventilator, der ein Atemgas mit einem von der Steuereinheit vorgegebenen Druck liefert. Mit Elektroden wird elektromyographisch ein elektrisches Signal aufgenommen und hieraus die Atemaktivität und der Atemmuskeldruck bestimmt.

Die WO 2006/131149 A1 offenbart ein System zum Beatmen eines Patienten mit einer Gasfördereinrichtung, einem Atemluftleitungssystem, einem EMG-Sensor zur nicht-invasiven Erfassung der elktromyographischen Muskelaktivität der Atemmuskulatur eines beatmeten Patienten, einer Steuereinrichtung zur Steuerung der Leistung der Gasfördereinrichtung in Abhängigkeit der erfassten Muskelaktivität, einer Haupteinheit, in der die Gasfördereinrichtung vorgesehen ist, und einem Messmodul, der getrennt von der Haupteinheit vorgesehen ist und der den EMG-Sensor aufweist.

Die AU 199672679 B2 offenbart ein System zur Beatmung von Patienten mit einer Gasfördereinrichtung, einer Gasleitung, einem nicht-invasiven EMG-Sensor und einer Steuereinrichtung zum Steuern der Gasfördereinrichtung in Abhängigkeit der Messdaten des EMG-Sensors. Außerdem offenbart D2 eine Analog-Digital-Wandeleinheit zur Umwandlung der von dem EMG-Sensor erfassten elektromyographischen Muskelaktivität in digitale Signale.

Die US 5,353,788 beschreibt ein System zur Beatmung eines Patienten mit einer Gasfördereinrichtung, einer Gasleitung, einem Sensor zur Erfassung der Muskelaktivität der Atemmuskulatur an der Hautoberfläche eines Patienten, einer Steuereinrichtung zur Steuerung der Gasfördereinrichtung in Abhängigkeit der gemessenen Muskelaktivität des Patienten, einer Haupteinheit, die die Gasfördereinrichtung aufweist, und mit einem Messmodul, der von der Haupteinheit getrennt ist, und den Sensor aufweist. Ferner beschreibt D3 eine Analog-Digital-Wandeleinheit zur Umwandlung der von dem Sensor erfassten Muskelaktivität des Patienten in digitale Signale sowie eine Batterie zur Versorgung des Messmoduls.

Die DA 101 64 446 A1 offenbart ein System zur Beatmung von Patienten, wobei der Druck des dem Patienten zugeführten Atemgases auf Basis der von einem EMG-Sensor gemessenen Muskelaktivität der Atemmuskulatur gesteuert wird.

Die DE 10 2007 062 214 B3. offenbart ein System zur Beatmung von Patienten mit einer Gasfördereinrichtung, einem EMG-Sensor zur Messung der Muskelaktivität der Atemmuskulatur eines Patienten sowie einer Steuereinrichtung zur Steuerung der Leistung der Gasfördereinrichtung in Abhängigkeit der Messdaten des EMG-Sensors.

Die US 2006/0152197 A1 offenbart ein System zur Versorgung einer elektronischen Vorrichtung mit elektrischer Leistung von wahlweise entweder einer Batterie oder einer externen Stromquelle.

Die Aufgabe der vorliegenden Erfindung besteht deshalb darin, ein Beatmungs- oder Anästhesiesystem zur Verfügung zu stellen, bei dem eine Anpassung des Anteils der Beatmung an die Leistungsfähigkeit der Atemmuskulatur des beatmeten Patienten ohne eine invasive Messung der elektromyographischen Aktivität der Atemmuskulatur möglich ist.

Diese Aufgabe wird gelöst mit einem Beatmungs- oder Anästhesiesystem zur Beatmung eines Patienten, umfassend eine Gasfördereinrichtung (3), wenigstens eine Gasleitung (4) zur Ausbildung eines Atemluftleitungssystems, in Form eines Atemluftkreissystems, wenigstens einen EMG-Sensor (6) zur nicht-invasiven Erfassung der elektromyographischen Muskelaktivität der Atemmuskulatur eines zu beatmenden Patienten (20) an dessen Hautoberfläche, eine Steuereinrichtung (9) zur Steuerung oder Regelung der Leistung der Gasfördereinrichtung (3) in Abhängigkeit von der erfassten Muskelaktivität der Atemmuskulatur, eine Haupteinheit (2), welche die Gasfördereinrichtung (3) aufweist, wenigstens ein Messmodul (5), an welchem der wenigstens eine EMG-Sensor (6) angeordnet ist und das eine von der Haupteinheit (2) getrennte tragbare Baueinheit ist, wobei das wenigstens eine Messmodul (5) mit einer Analog-Digital-Wandeleinheit (10) zur Umwandlung der von dem wenigstens einen EMG-Sensor (6) erfassten analogen elektronryographischen Muskelaktivität in digitale Signale versehen ist, wobei das wenigstens eine Messmodul (5) eine Energieversorgungseinheit in Form von wenigstens einer Batterie (13) und/oder wenigstens einem Kondensator aufweist, wobei die Haupteinheit (2) mit einer Ladungseinheit (14) für die wenigstens eine Batterie (13) und/oder den wenigstens einen Kondensator versehen ist, wobei in das Messmodul (5) ein Sender und in die Haupteinheit (2) ein Empfänger (22) zur drahtlosen Übertragung der digitalen Signale von dem Messmodul (5) zu der Haupteinheit (2) eingebaut sind. Ein sEMG-Sensor ist ein surface EMG-Sensor und erfasst die elektrische Aktivität der Atemmuskulatur nicht-invasiv, d. h. an der Oberfläche, insbesondere Hautoberfläche, des Patienten. Damit ist in vorteilhafter Weise eine für den Patienten unangenehme oder schädliche invasive Messung der elektrischen Aktivität der Atemmuskulatur, beispielsweise in der Luftröhre invasiv mit einem EMG-Sensor, nicht mehr erforderlich. Der Beginn der Atemphase des Patienten und/oder die Eigenatemaktivität bzw. Eigenatmung des Patienten kann damit nicht-invasiv mit dem wenigstens einen sEMG-Sensor erfasst werden.

In einer ergänzenden Variante ist das Beatmungs- oder Anästhesiesystem mit einer Einrichtung, z. B. wenigstens eine Taste und/oder ein Hebel, zur Steuerung und/oder Regelung und/oder Veränderung des Anteils der Beatmung versehen. Ein Arzt kann damit den Anteil der Beatmung erhöhen und reduzieren und damit an den Gesundheitszustand des Patienten anpassen, beispielsweise für eine langsame Entwöhnung des Patienten von der Beatmung. Eine Erhöhung der Beatmung bedingt eine Reduzierung der Eigenatmung des Patienten und umgekehrt.

Vorzugsweise umfasst das Beatmungs- oder Anästhesiesystem wenigstens zwei sEMG-Sensoren, insbesondere wenigstens ein Paar an sEMG-Sensoren.

Erfindungsgemäß umfasst das Beatmungs- oder Anästhesiesystem wenigstens ein Messmodul und der wenigstens eine sEMG-Sensor ist an dem wenigstens einen Messmodul angeordnet.

Vorzugsweise ist der wenigstens eine sEMG-Sensor in einem Abstand von weniger als 100 cm, 70 cm, 50 cm, 30 cm, 20 cm oder 10 cm zu dem Messmodul angeordnet.

In einer weiteren Ausgestaltung ist der wenigstens eine sEMG-Sensor mit einem Analogkabel oder mit einer Funkverbindung mit dem wenigstens einen Messmodul verbunden. Die von dem wenigstens einen sEMG-Sensor, an der Hautoberfläche gemessene, Spannung aus der Atemmuskulaturaktivität wird mit dem Analogkabel zu dem wenigstens einen Messmodul geleitet. Dabei werden sehr kleine Spannungen im Bereich von einigen bis ungefähr 100 µV von dem wenigstens einen sEMG-Sensor gemessen. Damit kann in dem wenigstens einen SEMG-Sensor die gemessene Spannung in digitale Signale umgewandelt werden und in das Messmodul und/oder die Haupteinheit übertragen werden.

Erfindungsgemäß weist das Beatmungs- oder Anästhesiesystem eine Haupteinheit mit der Gasfördereinrichtung und vorzugsweise einem Hauptgehäuse auf und das wenigstens eine Messmodul ist eine von der Haupteinheit getrennte, vorzugsweise tragbare, Baueinheit.

Erfindungsgemäß ist das wenigstens eine Messmodul mit einer Analog-Digital-Wandeleinheit zur Umwandlung der von dem wenigstens einen sEMG-Sensor erfassten analogen elektromyographischen Muskelaktivität in digitale Signale versehen. Der wenigstens eine sEMG-Sensor erfasst nur sehr kleine Spannungen aus der Atemmuskulatur, so dass bei einer Leitung dieser sehr kleinen Ströme über eine größere Entfernung zu der Haupteinheit diese sehr kleinen Spannungen verfälscht werden können. Aus diesem Grund ist eine Umwandlung dieser sehr kleinen gemessenen Spannungen in digitale Signale sinnvoll und wirkungsvoll bei einer örtlich nahen Umwandlung in der Analog-Digital-Wandeleinheit in dem wenigstens einen Messmodul.

Erfindungsgemäß weist das wenigstens eine Messmodul eine Energieversorgungseinheit und vorzugsweise eine Datenverarbeitungseinheit auf.

Erfindungsgemäß ist die Energieversorgungseinheit wenigstens eine Batterie, vorzugsweise mehrere Batterien, und/oder ein Kondensator, vorzugsweise mehrere Kondensatoren, als Energiespeicher.

Erfindungsgemäß ist die Haupteinheit mit einer Ladungseinheit für den wenigstens einen Energiespeicher versehen.

In einer ergänzenden Ausführungsform ist die Energieversorgungseinheit in dem wenigstens einen Messmodul wenigstens ein Stromkabel und das wenigstens eine Stromkabel ist zu der Haupteinheit geführt zum Leiten von elektrischem Strom von der Haupteinheit zu dem wenigstens einen Messmodul und vorzugsweise ist an der Haupteinheit der von dem wenigstens einen Stromkabel geleitete Strom mit einer Induktionseinheit mit zwei Spulen mittels Induktion von der Haupteinheit zu dem wenigstens einen Stromkabel leitbar. Der Strom wird in der Induktionseinheit von einer Primärspule mittels Induktion auf eine Sekundärspule übertragen und damit ist das Messmodul elektrisch und/oder magnetisch von der Haupteinheit entkoppelt.

Insbesondere ist die Induktionseinheit in oder an der Haupteinheit angeordnet.

In einer weiteren Ausführungsform ist zur Energieversorgung des wenigstens einen Messmoduls das Beatmungs- oder Anästhesiesystem mit wenigstens zwei Energiespeichern, insbesondere eine Batterie oder ein Kondensator, und einer Ladungs- und Schalteinheit versehen, sodass während einer Entladephase wenigstens einen Energiespeichers der wenigstens eine Energiespeicher von einer Energieversorgung der Haupteinheit elektrisch getrennt sowie mit dem Messmodul elektrisch verbunden ist und während einer Aufladephase des wenigstens einen Energiespeichers der wenigstens eine Energiespeicher elektrisch von dem wenigstens einem Messmodul getrennt ist sowie mit der Haupteinheit elektrisch verbunden ist. Damit ist das wenigstens eine Messmodul von der Haupteinheit elektrisch und/oder magnetisch entkoppelt, weil das wenigstens eine Messmodul nicht gleichzeitig mit einem elektrischen Kabel direkt mit der Haupteinheit verbunden ist.

In einer zusätzlichen Ausgestaltung ist das wenigstens eine Messmodul mit wenigstens einem MMG-Sensor (mechanomyographischer Sensor) und/oder wenigstens einem Beschleunigungssensor und/oder einem Mikrofon zur nicht-invasiven Erfassung der Muskelaktivität der Atemmuskulatur, so dass diese Sensoren nicht-invasive Sensoren sind. Der wenigstens eine MMG-Sensor (mechanomyographischer Sensor) und/oder der wenigstens einem Beschleunigungssensor und/oder das Mikrofon ist in analoger Weise wie der wenigstens eine sEMG-Sensor mit einem Analogkabel mit dem Messmodul verbunden.

Vorzugsweise ist von der Steuereinrichtung die Leistung der Gasfördereinrichtung und/oder der Druck des Inspirationsgases in Abhängigkeit von den Daten und/oder Strömen des wenigstens einem MMG-Sensors und/oder des wenigstens einen Beschleunigungssensors und/oder des Mikrofons steuerbar und/oder regelbar.

Bei einem Verfahren zur Steuerung und/oder Regelung eines Beatmungs- oder Anästhesiesystems, insbesondere eines in dieser Schutzrechtsanmeldung beschriebenen Beatmungs- oder Anästhesiesystems, können die Schritten vergesehen sein: Fördern von Atemluft durch wenigstens eine Gasleitung eines Atemluftleitungssystems mit einer Gasfördereinrichtung, Erfassen der elektromyographischen Muskelaktivität der Atemmuskulatur eines zu beatmenden Patienten vorzugsweise mit einem EMG-Sensor, Steuern und/oder Regeln der Leistung der Gasfördereinrichtung und/oder des Druckes der Inspirationsluft in Abhängigkeit von der erfassten Muskelaktivität der Atemmuskulatur, wobei die elektromyographische Muskelaktivität von wenigstens einem sEMG-Sensor an einer Hautoberfläche des zu beatmenden Patienten erfasst wird.

In einer zusätzlichen Ausgestaltung werden mit einer Analog-Digital-Wandeleinheit die von dem wenigstens einen sEMG-Sensor erfasste analoge elektromyographische Muskelaktivität in digitale Signale umgewandelt. Der sEMG-Sensor erfasst als analoge Muskelaktivität eine sehr kleine Spannung des Atemmuskels.

Zweckmäßig wird wenigstens ein Messmodul mit dem wenigstens einen sEMG-Sensor mit elektrischer Energie aus einer Haupteinheit des Beatmungs- oder Anästhesiergerätes versorgt.

In einer weiteren Ausgestaltung wird die elektrische Energie durch wenigstens ein Stromkabel von der Haupteinheit zu dem wenigstens einem Messmodul geleitet und dabei wird, vorzugsweise an der Haupteinheit, der Strom mit Induktion übertragen zur Verringerung der Koppelkapazität zwischen dem wenigstens einem Messmodul und der Haupteinheit (das wenigstens eine Messmodul ist damit elektrisch und/oder magnetisch von der Haupteinheit entkoppelt, weil eine Stromleitung von zu der Haupteinheit zu dem wenigstens einen Messmodul nur mittels Induktion möglich ist) und/oder an dem wenigstens einem Messmodul ist wenigstens ein Energiespeicher angeordnet und nach dem Erfassen der elektromyographischen Muskelaktivität mit dem wenigstens einem Messmodul an dem Patienten wird das wenigstens eine Messmodul zu der Haupteinheit gebracht und an der Haupteinheit wird der wenigstens eine Energiespeicher aufgeladen und vorzugsweise wird wenigstens ein anderes Messmodul nach dem Aufladen des wenigstens einen Energiespeichers an der Haupteinheit das wenigstens eine andere Messmodul zum Patienten gebracht zum Erfassen der elektromyographischen Muskelaktivität (das wenigstens eine Messmodul ist damit elektrisch und/oder magnetisch von der Haupteinheit entkoppelt, weil der Energiespeicher nur aufgeladen wird, wenn der Energiespeicher nicht mit dem Messmodul, sondern nur mit der Haupteinheit elektrisch verbunden ist) und/oder das wenigstens eine Messmodul wird mit wenigstens zwei Energiespeichern mit elektrischer Energie versorgt und während einer Entladephase wenigstens eines Energiespeichers wird das wenigstens eine Messmodul mit elektrischer Energie versorgt und während der Entladephase der wenigstens eine Energiespeicher elektrisch von der Haupteinheit getrennt wird und/oder während einer Aufladephase des wenigstens einen Energiespeichers der wenigstens eine Energiespeicher mit elektrischer Energie aus der Haupteinheit aufgeladen wird und während der Aufladephase der wenigstens eine Energiespeicher elektrisch von dem wenigstens einen Messmodul getrennt wird und/oder ist (das wenigstens eine Messmodul ist damit elektrisch und/oder magnetisch von der Haupteinheit entkoppelt, weil der Energiespeicher nur aufgeladen wird, wenn der Energiespeicher nicht mit dem Messmodul, sondern nur mit der Haupteinheit elektrisch verbunden ist).

In einer weiteren Ausgestaltung werden die digitalen Daten zur Muskelaktivität der Atemmuskulatur drahtlos (mit einer Funkverbindung) oder drahtgebunden von dem wenigstens einen Messmodul zu der Haupteinheit übertragen.

In einer weiteren Ausgestaltung ist eine Koppelkapazität zwischen dem wenigstens einem Messmodul und der Haupteinheit kleiner als 40 bis 50 pF, vorzugsweise kleiner als 30 bis 40 pF, insbesondere kleiner als 10 bis 12 pF (pFarad).

In einer ergänzenden Variante weist die Haupteinheit des Beatmungs- oder Anästhesiesystems einen CO₂-Absorber auf. Von dem CO₂-Absorber wird dem von dem zu beatmenden Patienten ausgeatmete Exspirationsgas das Kohlendioxid entzogen.

Zweckmäßig umfasst das Beamtungs- oder Anästhesiesystem einen Inspirations- und ein Exspirationsschlauch.

In einer weiteren Variante weist das Beatmungs- oder Anästhesiesystem ein Y-Stück oder eine Atemmaske auf.

In einer ergänzenden Variante sind der Inspirations- und/oder Exspirationsschlauch an dem Y-Stück oder der Atemmaske angeschlossen.

Vorzugsweise sind an dem Inspirations- und Exspirationsschlauch jeweils ein Rückschlagventil angeordnet.

In einer zusätzlichen Ausgestaltung weist die Haupteinheit des Beatmungs- oder Anästhesiesystem einen Anästhesiemittelreflektor auf. Mittels des Anästhesiemittelreflektors wird das Anästhesiemittel, z. B. Halotan oder Enfluran, dem Exspirationsgas entzogen und anschließend das entzogene Anästhesiemittel dem Inspirationsgas zugeführt.

In einer zusätzlichen Ausgestaltung weist die Haupteinheit des Beatmungs- oder Anästhesiesystems einen Anästhesiemitteldosierer mit einem Anästhesiemittelvorratsbehälter auf. Mittels des Anästhesiemitteldosierers wird dem Inspirationsgas Anästhesiemittel zugesetzt. Das Anästhesiemittel liegt dabei im Allgemeinen in einer flüssigen Form vor und das Anästhesiemittel wird in dem Anästhesiemitteldosierer von der flüssigen in die gasförmige Phase umgewandelt und somit dem Patienten zugeführt.

Bei der Ausbildung eines Atemluftkreissystems wird das von dem Patienten ausgeatmete Exspirationsgas wenigstens teilweise wieder als Inspirationsgas dem Patienten zugeführt. Dabei wird dem ausgeatmeten Exspirationsgas, vorzugsweise in dem CO₂-Absorber, das Kohlendioxid entzogen und vorzugsweise wird in einem Gasmischer das dem Patienten zuzuführende Inspirationsgas mit einer Mischung aus Sauerstoff, Lachgas, Luft und/oder Xenon angereichert.

Im Nachfolgenden werden Ausführungsbeispiele der Erfindung unter Bezugnahme auf die beigefügte Zeichnung näher beschrieben.

Es zeigen:
- Fig. 1: eine stark vereinfachte Darstellung eines Beatmungs- oder Anästhesiesystems in einem ersten Ausführungsbeispiel,
- Fig. 2: eine stark vereinfachte Darstellung des Beatmungs- oder Anästhesiesystems in einem zweiten Ausführungsbeispiel,
- Fig. 3: eine stark vereinfachte Darstellung des Beatmungs- oder Anästhesiesystems in einem dritten Ausführungsbeispiel,
- Fig. 4: einen Parkhalter in einem ersten Ausführungsbeispiel des Beatmungs- oder Anästhesiesystems gemäß Fig. 3,
- Fig. 5: den Parkhalter in einem zweiten Ausführungsbeispiel des Beatmungs- oder Anästhesiesystems gemäß Fig. 3,
- Fig. 6: eine Ladungs- und Schalteinheit des Beatmungs- und Anästhesiegerätes gemäß Fig. 7 und
- Fig. 7: eine stark vereinfachte Darstellung des Beatmungs- oder Anästhesiesystems in einem vierten Ausführungsbeispiel.

Beatmungsgeräte bzw. Beatmungssysteme 1 werden zur Beatmung von Patienten 20 und Anästhesiegeräte bzw. Anästhesiesysteme 1 werden neben der Beatmung auch zur Narkose von Patienten 20 eingesetzt. Das Beatmungs- oder Anästhesiesystem 1 weist dabei einen Atemluftkreissystem auf, d. h., dass das von dem Patienten 20 ausgeatmete Exspirationsgas für eine Rückatmung als Inspirationsgas wiederverwendet wird. Die Atemluft wird dabei von einer Gasfördereinrichtung 3 als Gebläse in einer Haupteinheit 2 durch Gasleitungen 4 in einem Atemluftkreissystem geleitet. Die Gasleitungen 4 sind somit an die Haupteinheit 2 angeschlossen. In den Gasleitungen 4 sind ein erstes, inspiratorisches Rückschlagventil und ein zweites expiratorisches Rückschlagventil angeordnet. Dadurch bildet sich eine Exspirationsgasleitung bzw. ein Exspirationsschlauch 8 und eine Inspirationsgasleitung bzw. ein Inspirationsschlauch 7 aus. Am Ende der Inspirations- und Exspirationsgasleitung 7, 8 ist ein Y-Stück angeschlossen, das mittels eines Patientenanschlussstückes das Inspirations- und Exspirationsgas zu und von einem zu beatmenden Patienten 20 leitet. Ein nicht dargestellter CO₂-Absorber in der Haupteinheit 2 absorbiert das in dem Exspirationsgas enthaltene Kohlendioxid. Ferner wird mit einem nicht dargestellten Anästhesiemittelreflektor in der Haupteinheit 2 und einem Anästhesiemitteldosierer das Inspirationsgas mit Anästhesiemittel angereichert (nicht dargestellt). Zusätzlich wird mittels eines nicht dargestellten Gasmischers in der Haupteinheit dem Inspirationsgas eine Mischung aus Sauerstoff und Lachgas zugeführt. Dem Gasmischer wird dabei mittels zweier Ventile separat der Sauerstoff und das Lachgas zugeführt.

Das Beatmungs- oder Anästhesiesystem 1 umfasst ferner ein Messmodul 5 mit zwei sEMG-Sensoren 6, wobei mit einem Analogkabel von geringer Länge, beispielsweise im Bereich zwischen 3 und 15 cm, die sEMG-Sensoren 6 elektrisch mit einem Messmodul 5 verbunden sind. Die beiden sEMG-Sensoren 6 liegen im Brustbereich auf der Haut des Patienten 20 auf, d. h. von den sEMG-Sensoren 6 wird die elektrische Aktivität der Atemmuskulatur nicht-invasiv gemessen. Das Messmodul 5 verfügt über nicht dargestellte Steckverbinder zum Anschließen von bis zu fünf Paaren an sEMG-Sensoren 6 (nicht dargestellt). Innerhalb eines Gehäuses (nicht dargestellt) des Messmoduls 5 ist eine Analog-Digital-Wandeleinheit 10 und eine Datenverarbeitungseinheit 11 angerordnet. Die sEMG-Sensoren 6 messen sehr kleine Ströme mit einer Spannung zwischen einigen bis ungefähr 100 µV, so dass bei einer Leitung dieser geringen Ströme über eine größere Entfernung durch ein langes Analogkabel eine Verfälschung dieser kleinen gemessenen Spannungen eintreten kann. Aus diesem Grund werden mit der Analog-Digital-Wandeleinheit 10 die von den beiden sEMG-Sensoren 6 erfassten Spannungen in einer geringen Entfernung von den sEMG-Sensoren 6 in digitale Signale umgewandelt. Das Messmodul 5 wird durch zwei Stromkabel 15 (nur ein Stromkabel 15 dargestellt) mit elektrischen Strom aus der Haupteinheit 2 versorgt. Die Stromkabel 15 in dem Messmodul 5 stellen damit eine Energieversorgungseinheit (nicht dargestellt) dar. In der Haupteinheit 2 ist ein DC-DC-Wandler 17 als Induktionseinheit 19 angeordnet mit einer nicht dargestellten Primär- und Sekundärspule. Dabei wird Gleichstrom zunächst moduliert, anschließend der modulierte Gleichstrom mittels Induktion von der Primär- auf die Sekundärspule übertragen und anschließend der in der Sekundärspule induzierte Strom wieder in Gleichstrom umgewandelt und durch die beiden Stromkabel 15 zu dem Messmodul 2 geleitet zur Stromversorgung des Messmoduls 2. Damit ist die Stromversorgung des Messmoduls 2 elektrisch bzw. magnetisch von der Haupteinheit 2 entkoppelt, so dass Störungen bei der Wandlung der gemessenen analogen Ströme in digitale Signale in der Analog-Digital-Wandeleinheit 10 sehr gering ausfallen.

Das Messmodul 5 ist mit einem Datenkabel 16 mit der Haupteinheit 2 verbunden zur Übertragung der von der Analog-Digital-Wandeleinheit 10 erzeugten digitalen Daten in die Haupteinheit 2. Dabei ist das Datenkabel 16 an eine Potentialtrenneinheit 18 in der Haupteinheit 2 angeschlossenen und von der Potentialtrenneinheit 18 werden die digitalen Daten zu einer Steuereinrichtung 9 in der Haupteinheit 2 übertragen. Das Datenkabel 16 kann ein elektrisches Kabel zur Leitung von Strom oder ein Glasfaserkabel zur optischen Übertragung von Daten sein. Mit der Potentialtrenneinheit 18 wird das elektrische Potential des Messmoduls 5 von dem elektrischen Potential der Haupteinheit 2 getrennt, damit auch bei unterschiedlichen elektrischen Potentialen in der Haupteinheit 2 und dem Messmodul 5 durch das Datenkabel 16 kein hieraus resultierender Strom fließt. In der Steuereinrichtung 9 werden die digitalen Signale ausgewertet und in Abhängigkeit von den digitalen Signalen, d. h. der von den beiden sEMG-Sensoren 6 auf der Hautoberfläche gemessenen elektrischen Potentialdifferenz aus der Atemmuskelaktivität, die Leistung der Gasfördereinrichtung 3 gesteuert und/oder geregelt.

In der Datenverarbeitungseinheit 11 können digitale Signale gespeichert und verarbeitet werden und ferner kann ein Messmodul 5 und damit auch ein Patient 20 von der Steuerungseinheit 9 identifiziert werden bei einer Verwendung von mehreren Messmodulen 5 für die gleiche Haupteinheit 2. Außerdem können von der Datenverarbeitungseinheit 11 Checksummen der übertragenen digitalen Daten und/oder Kontrollsignale zu der Steuereinrichtung 9 übertragen werden, um mögliche Fehler bei der Übertragung von dem Messmodul 5 zu der Haupteinheit 2 bzw. der Steuereinrichtung 9 zu erfassen. Damit soll eine falsche Beatmung des Patienten 20 aufgrund von Datenübertragungsfehlern ausgeschlossen werden.

Das Messmodul 5 weist außerdem wenigstens einen Sensor (nicht dargestellt) zur Erfassung eines Patientengrounds, d. h. des durchschnittlichen elektrischen Potentials des Patienten 20, auf. Abweichend hiervon kann der Patientenground auch aus dem von den sEMG-Sensoren 6 erfassten durchschnittlichen elektrischen Potential berechnet werden. Es handelt sich bei diesem Sensor um einen nicht-invasiven Sensor, der das elektrische Potential an der Hautoberfläche misst. Das elektrische Potential in dem Messmodul 5 wird mit der Datenverarbeitungseinheit 11 derart gesteuert, dass das elektrische Potential in dem Messmodul 5 im Wesentlichen dem Patientenground entspricht, um hieraus resultierende Ströme in dem Analogkabel zur Verbindung der sEMG-Sensoren 6 mit dem Messmodul 5 zu verhindern.

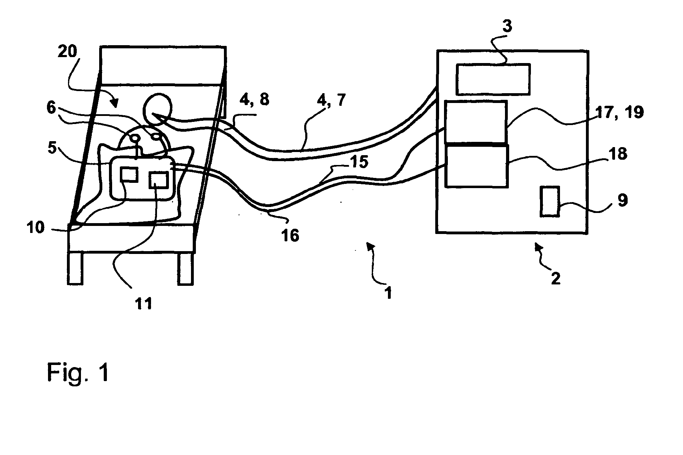

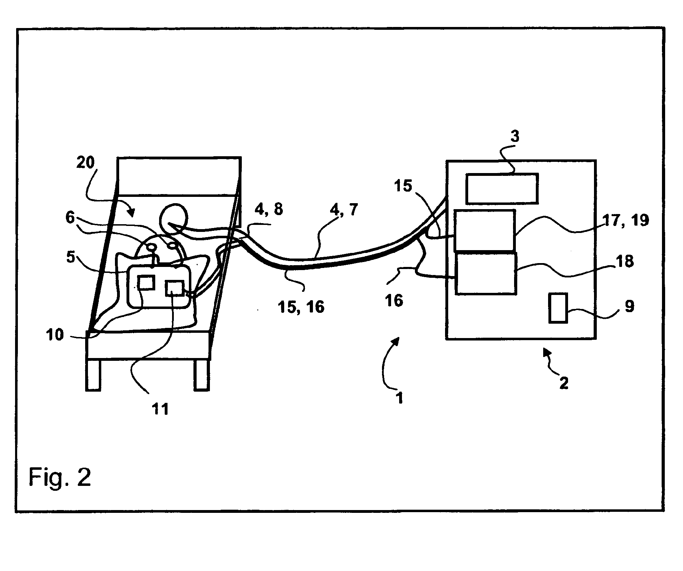

In Fig. 2 ist ein zweites Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. Das Stromkabel 15 und das Datenkabel 16 zur Verbindung des Messmoduls 5 mit der Haupteinheit 2 ist in den Inspirationsschlauch 7 und/oder den Exspirationsschlauch 8 integriert, so dass die Haupteinheit 2 und das Messmodul 5 nur mit eine Leitungseinheit bzw. einem Kabelkanal miteinander verbunden sind sowohl zur pneumatischen als auch zur elektrischen Verbindung.

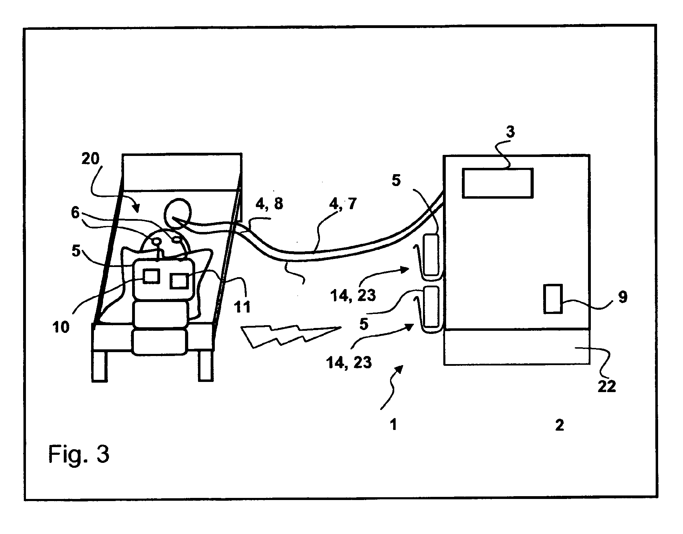

In Fig. 3 ist ein drittes Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. In das Messmodul 5 ist eine Batterie 13 als Energiespeicher 12 eingebaut und dient zur elektrischen Energieversorgung des Messmoduls 5. Außerdem ist in das Messmodul 5 ein Sender 21 und in die Haupteinheit 2 ein Empfänger 22 eingebaut zur drahtlosen Übertragung der digitalen Signale von dem Messmodul 5 zu der Haupteinheit 2 bzw. der Steuereinrichtung 9. Von dem Empfänger 22 werden die digitalen Signale bezüglich der elektromyographischen Atemmuskelaktivität zu der Steuereinrichtung 9 übertragen. Dabei kann von der Steuereinrichtung 9 ein Messmodul 5 identifiziert werden mit einer bidirektionalen Funkverbindung.

Das Beatmungs- oder Anästhesiesystem 1 umfasst zwei oder drei Messmodule 5, weil sich ein Messmodul 5 beim Patienten 5 befindet zur Erfassung der elektromyographischen Atemmuskelaktivität und Übertragung der digitalen Daten, so dass die Batterie 13 in dem Messmodul 5 am Patienten 5 entladen wird. Das zweite oder dritte Messmodul 5 ist in einer Ladungseinheit 14 als Parkhalter 23 angeordnet und wird aufgeladen. Das Messmodul 5 an dem Patienten 20 wird bei einem Unterschreiten eines vorgegebenen Ladezustandes der Batterie 13 durch ein in der Ladungseinheit 14 aufgeladenes Messmodul 5 ersetzt. Dabei ist das Messmodul 5 mit einer nicht dargestellten zentralen Steckeinheit versehen, mit dem sämtliche an dem Patienten 20 angeordnete sEMG-Sensoren 6 mit dem Messmodul 5 verbunden werden können. Damit besteht auch keine direkte elektrische Verbindung zwischen der Haupteinheit 2 und dem Messmodul 5 am Patienten 20, so dass damit das Messmodul 5 an dem Patienten 20 elektrisch und magnetisch von der Haupteinheit 2 entkoppelt ist. Die Aufladezeit eines Messmoduls 5 in dem Parkhalter 23 beträgt maximal die Hälfte der Betriebsdauer des Messmoduls 5 am Patienten. Zweckmäßig beträgt die Aufladezeit 1/4 oder 1/6 der Betriebsdauer. Eine Betriebsdauer von 24 h steht z. B. eine Aufladezeit von 4 h gegenüber. Während der Aufladens des Messmoduls 5 können auch digitale Daten mittels nicht dargestellter Kontaktelemente von dem Messmodul 5 in die Steuereinrichtung 9 übertragen werden, beispielsweise bezüglich der Historie des Messmoduls 5, der Ladezyklen, der Betriebsstunden und Statusinformationen. Das Messmodul 5 kann von der Steuereinrichtung 9 auch identifiziert werden mittels Daten in der Datenverarbeitungseinheit 11.

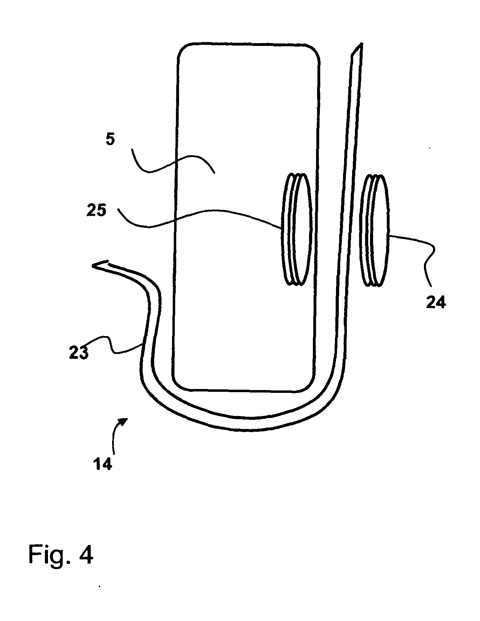

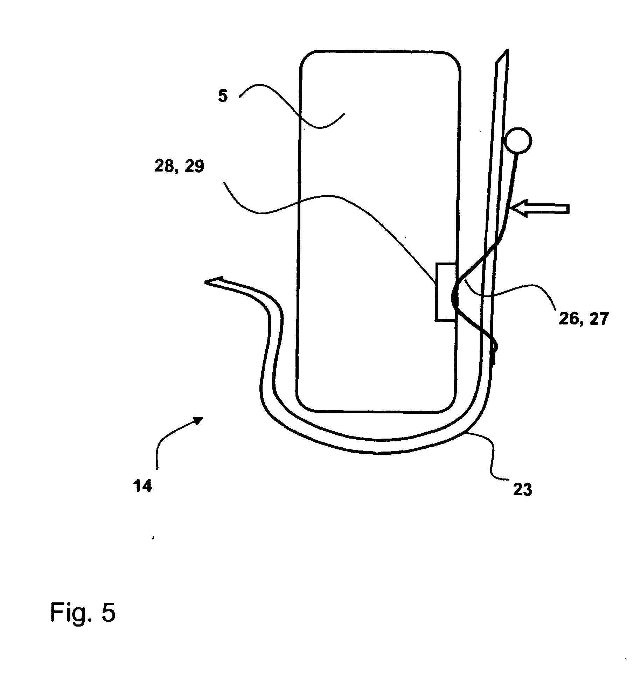

Fig. 4 und 5 zeigen zwei Ausführungsbeispiele für die Ladungseinheit 14. Im ersten Ausführungsbeispiel gemäß Fig. 1 erfolgt die Energie- bzw. Stromübertragung von einer Geberspule 24 an der Haupteinheit 2 mittels elektrischer Induktion kontaktlos zu einer Nehmerspule 25 in dem Messmodul 5. Das zweite Ausführungsbeispiel (Fig. 5) der Ladungseinheit 14 zeigt eine kontaktgebundene Stromübertragung von der Haupteinheit 2 mit einem als Metallfeder 27 ausgebildeten Kontaktelement 26 an der Haupteinheit 2 und einem Metallplättchen 29 als Gegenkontaktelement 28. Die Ladungseinheit 2 weist je zwei Kontaktelemente 26 und das Messmodul 5 ist mit zwei Gegenkontaktelementen 28 versehen. Damit kann elektrischer Strom von der Haupteinheit 2 in die Batterie 13 geleitet werden.

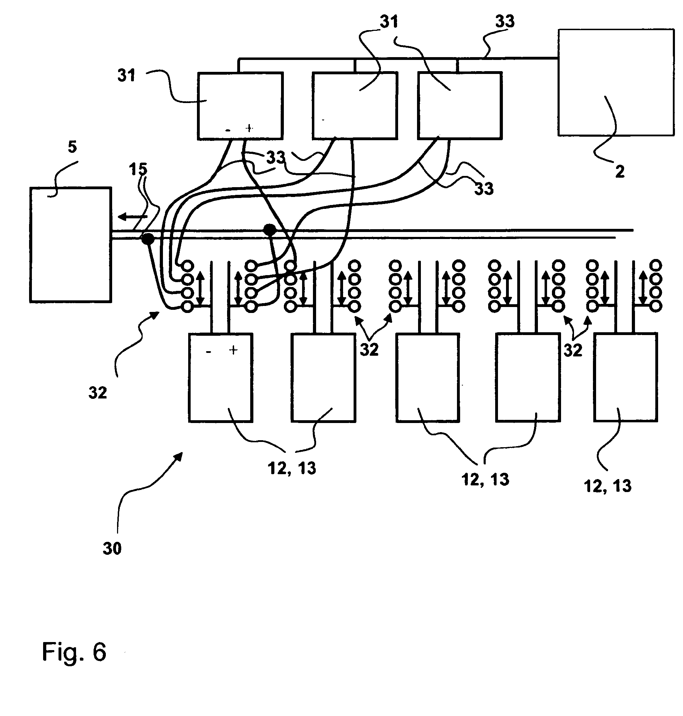

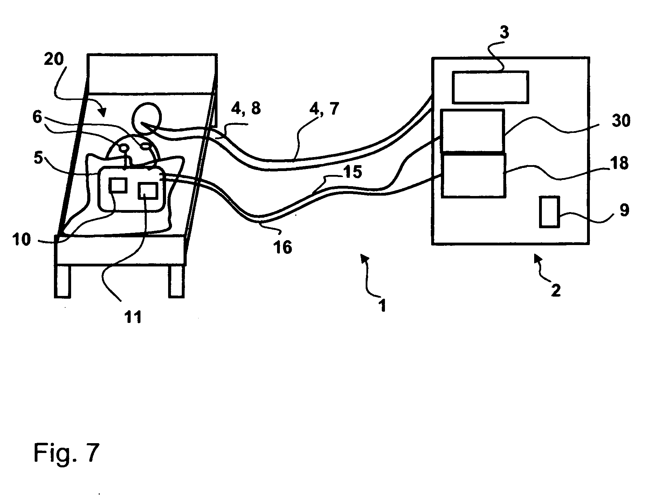

In Fig. 6 und 7 ist ein viertes Ausführungsbeispiel des Beatmungs- oder Anästhesiesystems 1 dargestellt. Im Nachfolgenden werden im Wesentlichen nur die Unterschiede zu dem ersten Ausführungsbeispiel gemäß Fig. 1 beschrieben. In der Haupteinheit 2 ist eine Ladungs- und Schaleinheit 30 angeordnet. In der Ladungs- und Schalteinheit 30 werden Batterien 13 mit Ladungsteilen 31 aufgeladen. Die Ladungsteile 31 sind mit Ladungskabel 33 mit der Haupteinheit 2 und den Batterien 13 verbunden. Außerdem sind zu den Batterien 13 die Stromkabel 15 geführt, mit denen der Strom aus den Batterien 13 in das Messmodul 5 geleitet wird. Schalteinheiten 32 an den Batterien 13 dienen dazu, dass nur eine Batterie 13 mit dem Messmodul 5 verbunden ist zur Stromversorgung des Messmoduls 5 mit der einen Batterie 13 und wenigstens eine andere Batterie 13 nur mit einem Ladungsteil 31 verbunden ist zum Aufladen der wenigstens einen anderen Batterie 13. Nach dem Entladen einer Batterie 13 wird die entladene Batterie 13 mit der Schalteinheit 32 von dem Messmodul 5 elektrisch getrennt und eine andere aufgeladene Batterie 13 mit dem Messmodul 5 elektrisch verbunden. Die entladene Batterie 13 wird anschließend mit dem Ladungsteil 31 elektrisch verbunden und aufgeladen. Die Batterien 13 sind damit niemals zeitglich sowohl mit dem Messmodul 5 als auch mit dem Ladungsteil 31 bzw. der Haupteinheit 2 elektrisch verbunden, so dass das Messmodul 5 elektrisch bzw. magnetisch von der Haupteinheit 2 entkoppelt ist. Die Ladungs- und Schalteinheit 30 kann anstelle in der Haupteinheit 2 auch in dem Messmodul 5 angeordnet sein (nicht dargestellt).

Insgesamt betrachtet sind mit dem erfindungsgemäßen Beatmungs- oder Anästhesiesystem 1 wesentliche Vorteile verbunden. Die Gasfördereinrichtung 3 wird in Abhängigkeit von der elektromyographischen Atemmuskelaktivität betrieben, welche mit sEMG-Sensoren 6 gemessen wird, so dass eine nachteilige invasive Messung nicht mehr erforderlich ist. Die Energieversorgung des Messmoduls 5 ist elektrisch und magnetisch von der Haupteinheit 2 entkoppelt, so dass keine Störungen wegen einer elektrischen und magnetischen Koppelung des Messmoduls 5 mit der Haupteinheit 2 auftreten.

### BEZUGSZEICHENLISTE

- 1: Beatmungs- oder Anästhesiesystem
- 2: Haupteinheit
- 3: Gasfördereinrichtung
- 4: Gasleitung
- 5: Messmodul
- 6: sEMG-Sensor
- 7: Inspirationsschlauch
- 8: Exspirationsschlauch
- 9: Steuereinrichtung
- 10: Analog-Digital-Wandeleinheit
- 11: Datenverarbeitungseinheit
- 12: Energiespeicher
- 13: Batterie
- 14: Ladungseinheit
- 15: Stromkabel
- 16: Datenkabel
- 17: DC-DC-Wandler
- 18: Potentialtrenneinheit
- 19: Induktionseinheit
- 20: Patient
- 21: Sender
- 22: Empfänger
- 23: Parkhalter
- 24: Geberspule
- 25: Nehmerspule
- 26: Kontaktelement
- 27: Metallfeder
- 28: Gegenkontaktelement
- 29: Metallplättchen
- 30: Ladungs- und Schalteinheit
- 31: Ladungsteil
- 32: Schalteinheit
- 33: Ladungskabel

## Patentansprüche

1. Beatmungs- oder Anästhesiesystem zur Beatmung eines Patienten, umfassend
- eine Gasfördereinrichtung (3),
- wenigstens eine Gasleitung (4) zur Ausbildung eines Atemluftleitungssystems,
- wenigstens einen EMG-Sensor (6) zur nicht-invasiven Erfassung der elektromyographischen Muskelaktivität der Atemmuskulatur eines zu beatmenden Patienten (20) an dessen Hautoberfläche,
- eine Steuereinrichtung (9) zur Steuerung oder Regelung der Leistung der Gasfördereinrichtung (3) in Abhängigkeit von der erfassten Muskelaktivität der Atemmuskulatur,
- eine Haupteinheit (2), welche die Gasfördereinrichtung (3) aufweist,
- wenigstens ein Messmodul (5), an welchem der wenigstens eine EMG-Sensor (6) angeordnet ist und das eine von der Haupteinheit (2) getrennte tragbare Baueinheit ist,
wobei das wenigstens eine Messmodul (5) mit einer Analog-Digital-Wandeleinheit (10) zur Umwandlung der von dem wenigstens einen EMG-Sensor (6) erfassten analogen elektromyographischen Muskelaktivität in digitale Signale versehen ist,
wobei das wenigstens eine Messmodul (5) eine Energieversorgungseinheit in Form von wenigstens einer Batterie (13) und/oder wenigstens einem Kondensator aufweist,
**dadurch gekennzeichnet,**
**dass** die Haupteinheit (2) mit einer Ladungseinheit (14) für die wenigstens eine Batterie (13) und/oder den wenigstens einen Kondensator versehen ist,
**dass** in das Messmodul (5) ein Sender und in die Haupteinheit (2) ein Empfänger (22) zur drahtlosen Übertragung der digitalen Signale von dem Messmodul (5) zu der Haupteinheit (2) eingebaut sind.

2. Beatmungs- oder Anästhesiesystem nach Anspruch 1, **dadurch gekennzeichnet, dass** der Empfänger (22) eingerichtet ist, um digitale Signale zu der Steuereinrichtung (9) zu übertragen, dass zwischen Sender und Empfänger (22) eine bidirektionale Funkverbindung vorgesehen ist und dass die Steuereinrichtung (9) eingerichtet ist, um über die bidirektionale Funkverbindung ein Messmodul (5) zu identifizieren.

3. Beatmungs- oder Anästhesiesystem nach einem oder mehreren der Ansprüche 1 oder 2, **dadurch gekennzeichnet, dass** das wenigstens eine Messmodul (5) eine Datenverarbeitungseinheit (11) aufweist.

4. Beatmungs- oder Anästhesiesystem nach einem oder mehreren der Ansprüche 1 bis 3, **dadurch gekennzeichnet, dass** zur Energieversorgung des wenigstens einen Messmoduls (5) das Beatmungs- oder Anästhesiesystem mit wenigstens zwei Energiespeichern (12), insbesondere eine Batterie (13) oder ein Kondensator, und einer Ladungs- und Schalteinheit (30) versehen ist, sodass während einer Entladephase wenigstens einen Energiespeichers (12) der wenigstens eine Energiespeicher (12) von einer Energieversorgung der Haupteinheit (2) elektrisch getrennt sowie mit dem Messmodul (5) elektrisch verbunden ist und während einer Aufladephase des wenigstens einen Energiespeichers (12) der wenigstens eine Energiespeicher (12) elektrisch von dem wenigstens einen Messmodul (5) getrennt ist sowie mit der Haupteinheit (2) elektrisch verbunden ist.

5. Beatmungs- oder Anästhesiesystem nach einem oder mehreren der vorhergehenden Ansprüche, **dadurch gekennzeichnet, dass** das wenigstens eine Messmodul (5) mit wenigstens einem MMG-Sensor oder wenigstens einem Beschleunigungssensor oder einem Mikrofon versehen ist zur Erfassung der Muskelaktivität der Atemmuskulatur.

6. Beatmungs- oder Anästhesiesystem nach Anspruch 1, **dadurch gekennzeichnet, dass** das Atemluftleitungssystem als Atemluftkreissystem ausgebildet ist.

## Claims

1. Respirator or anesthesia system for respirating a patient comprising:
- a gas delivery means (3);
- at least one gas line (4) for forming a breathing air line system;
- at least one EMG sensor (6) for non-invasively detecting on the skin surface the electromyographic muscle activity of the respiratory muscles of a patient (20) to be respirated;
- a control unit (9) for controlling or regulating the output of the gas delivery means (3) in dependence on the detected muscle activity of the respiratory muscles;
- a main unit (2) including the gas delivery means (3);
- at least one measuring module (5) on which the at least one EMG sensor (6) is arranged, and which measuring module is a portable assembly unit separate from the main unit (2),
wherein the at least one measuring module (5) is provided with an analog-digital conversion unit (10) for converting analog electromyographic muscle activity detected by the at least one EMG sensor (6) into digital signals,
wherein the at least on measuring module (5) has an energy supply unit in the form of at least one battery (13) and/or at least one capacitor,
**characterized in that**
the main unit (2) is provided with a charging unit (14) for the at least one battery (13) and /or the at least one capacitor,
a transmitter is installed in the measuring module (5), and a receiver (22) is installed in the main unit (2), for wireless transmission of the digital signals from the measuring module (5) to the main unit (2).

2. Respirator or anesthesia system according to claim 1, **characterized in that** the receiver (22) is arranged to transmit digital signals to the control unit (9), **in that** between transmitter and receiver (22) a bidirectional radio connection is provided, and **in that** the control unit (9) is arranged to identify a measuring module (5) via the bidirectional radio connection.

3. Respirator or anesthesia system according one or more of the claims 1 or 2, **characterized in that** the at least one measuring module (5) comprises a data processing unit (11).

4. Respiratory or anesthesia system according to one or more of the claims 1 to 3, **characterized in that** the respiratory or anesthesia system is, to supply energy to the at least measuring module (5), provided with at least two energy storages (12), in particular a battery (13) or a capacitor, and with a charging and switching unit (30), so that during a discharge phase of at least one energy storage (12) the at least one energy storage (12) is electrically separated from an energy supply of the main unit (2) and is electrically connected to the measuring module (5), and during a charging phase of the at least one energy storage (12) the at least one energy storage (12) is electrically separated form the at least one measuring module (5) and is electrically connected to the main unit (2).

5. Respiratory or anesthesia system according to one or more of the preceding claims, **characterized in that** the at least one measuring module (5) is provided with at least one MMG sensor or at least one acceleration sensor or a microphone for detecting the muscle activity of the respiratory muscles.

6. Respirator or anesthesia system according to claim 1, **characterized in that** the breathing air line system is arranged in the form of a breathing air circulation system.

## Revendications

1. Système de ventilation ou d'anesthésie destiné à ventiler un patient, comprenant
- un dispositif de circulation de gaz (3),
- au moins une conduite de gaz (4) pour constituer un système de conduites d'air de respiration,
- au moins un capteur EMG (6) pour l'enregistrement non invasif de l'activité musculaire électromyographique des muscles respiratoires d'un patient (20) à ventiler, sur la surface de la peau de celui-ci,
- un dispositif de commande (9) pour commander ou réguler le débit du dispositif de circulation de gaz (3) en fonction de l'activité musculaire enregistrée des muscles respiratoires,
- une unité principale (2) qui présente le dispositif de circulation de gaz (3),
- au moins un module de mesure (5), sur lequel est disposé le capteur EMG (6), au nombre d'au moins un, et qui est une unité de construction portable, séparée de l'unité principale (2),
le module de mesure (5), au nombre d'au moins un, étant doté d'une unité de conversion analogique-numérique (10) destinée à convertir en signaux numériques l'activité musculaire électromyographique analogique, enregistrée par le capteur EMG, au nombre d'au moins un,
le module de mesure (5), au nombre d'au moins un, présentant une unité d'alimentation en énergie sous la forme d'au moins une batterie (13) et/ou d'au moins un condensateur,
**caractérisé**
**en ce que** l'unité principale (2) est dotée d'une unité de charge (14) pour la batterie (13), au nombre d'au moins une, et/ou le condensateur, au nombre d'au moins un,
**en ce qu'**un émetteur est monté dans le module de mesure (5) et un récepteur (22) est monté dans l'unité principale (2), en vue de la transmission sans fil des signaux numériques du module de mesure (5) à l'unité principale (2).

2. Système de ventilation ou d'anesthésie selon la revendication 1, **caractérisé en ce que** le récepteur (22) est conçu pour transmettre des signaux numériques au dispositif de commande (9), **en ce qu'**il est prévu une liaison radioélectrique bidirectionnelle entre l'émetteur et le récepteur (22), et **en ce que** le dispositif de commande (9) est conçu pour identifier un module de mesure (5) par l'intermédiaire de la liaison radioélectrique bidirectionnelle.

3. Système de ventilation ou d'anesthésie selon une ou plusieurs des revendications 1 ou 2, **caractérisé en ce que** le module de mesure (5), au nombre d'au moins un, présente une unité de traitement de données (11).

4. Système de ventilation ou d'anesthésie selon une ou plusieurs des revendications 1 à 3, **caractérisé en ce que**, pour l'alimentation en énergie du module de mesure (5), au nombre d'au moins un, le système de ventilation ou d'anesthésie est doté d'au moins deux accumulateurs d'énergie (12), en particulier une batterie (13) ou un condensateur, et d'une unité de charge et de commutation (30), de sorte que pendant une phase de décharge d'au moins un accumulateur d'énergie (12), l'accumulateur d'énergie (12), au nombre d'au moins un, est séparé électriquement d'une alimentation en énergie de l'unité principale (2) et est relié électriquement au module de mesure (5), et pendant une phase de charge de l'accumulateur d'énergie (12), au nombre d'au moins un, l'accumulateur d'énergie (12), au nombre d'au moins un, est séparé électriquement du module de mesure (5), au nombre d'au moins un, et est relié électriquement à l'unité principale (2).

5. Système de ventilation ou d'anesthésie selon une ou plusieurs des revendications précédentes, **caractérisé en ce que** le module de mesure (5), au nombre d'au moins un, est doté d'au moins un capteur MMG ou d'au moins un capteur d'accélération ou d'un microphone, afin d'enregistrer l'activité musculaire des muscles respiratoires.

6. Système de ventilation ou d'anesthésie selon la revendication 1, **caractérisé en ce que** le système de conduites d'air de respiration est constitué sous la forme d'un système de circuit d'air de respiration.
